# EUROPEAN PATENT APPLICATION

(11) **EP 1 714 623 A2**
(43) Date of publication of application: **25.10.2006**
(21) Application number: 06005389.9
(22) Date of filing: 16.03.2006
(51) Int. Cl.: A61F 5/01

(54) **A dynamic ankle orthesis**

(30) Priority: 22.04.2005 EP 05447088
(71) Applicant: Ortec N.V., B-3001 Leuven (BE)
(72) Inventor: Devreese, Serge Lucien Pierre Michel, B-4910 Theux (BE)
(74) Representative: Bird, William Edward

(57) **Abstract**

A dynamic ankle orthesis as subject of the present invention comprises a shoe insert (1) having a heel, a cuff (2) and a springy means (3, 6) for coupling the shoe insert (1) and the cuff (2). The springy means is coupled at its lower part (6) to the shoe insert (1) by means of a pivot (4) located at the heel of the shoe insert (1). The orthesis according to the present invention allows control and assistance of the foot during rotation around each of the three axes of rotation of the foot and heel in a distinct way during each of the phases of a step.

## Description

### Technical field of the invention

The present invention relates to a dynamic ankle orthesis, especially an orthesis for maintaining the dynamical behaviour of the combination of foot-ankle-leg, and for assisting and/or correcting neuromuscular deficiencies related to the three rotation axes of the foot. Rotations corresponding to these three rotation axes are a rotation around the vertical axis of the leg, stretching and bending of the ankle around the axis of the ankle and supination/pronation, this is rotation around the longitudinal axis of the foot.

### Background of the invention

A normal step of a person comprises several consecutive phases. In a first phase, called "heel-strike phase", the heel is placed on the ground under a slight supination, this is the foot being slightly inclined to the exterior side of the foot. The contact of the foot with the ground is made at the external back part of the heel.

In a second phase, called "mid-stance phase", the sole of the foot is brought in contact with the ground. The foot is canted progressively forward meanwhile making a pronation, this is rotating the foot to the internal side of the foot around the longitudinal axis of the foot. This is done in preparation of the third phase, called "toe-off phase", during which the foot is pushed off at the metatarsus of the big toe. After this toe-off phase, there is a fourth phase during which the leg is stretched and the foot is swung forwards. This phase is called the swing-phase. After a swing-phase, again a heel-strike phase is exercised.

Some patients suffer from pathology like multiple sclerosis, hemiplegics, myopathy or traumatic repercussions, generating neuromuscular problems, causing an insufficient control of positioning, and maintaining of its position, of the foot and the leg during walking. In order to obtain a stabilised walk, a good neuromuscular coordination is necessary, to obtain a correct foot contact with and positioning on the ground, which is continuously changing during each phase of a step.

Existing textile ankle supports are of little or no use for this type of pathology.

Other light apparatuses consist of a leaf spring and an upholstery or semi-rigid shell, which only provided a weak support to the foot.

The majority of ortheses for providing a strong foot support are rigid or may provide an articulation around the axis of the ankle, possibly with a system of abutment points or with articulating assistance as described in JP9206347.

The articulation may be provided by a leaf spring which has a dynamical effect, as described in WO 02/096328.

In 1965, Freeman et al. first postulated and provided evidence that a proprioceptive defect, e.g. after ankle sprain, caused functional instability of the joint. Freeman et al. explained that ligaments contain nerve endings that inform the brain of joint position. Proprioceptive awareness is important in preventing re-injury by providing good sensory feedback. Immobilization of the joint results in deterioration of proprioceptive function. More generally, and not limited to physical injury such as a sprain, the non-respecting of the mobility of the ankle according to all three rotation axes, generates a loss of proprioception, this is a loss of perception of the contact of foot with the ground, which perception is to be communicated to the brain for dosing the nervous influx to the muscle or group of muscles. In addition, this loss of perception, combined with a stringent positioning of the foot wearing the orthesis, may cause spasms and unbalancing of the patient.

An orthesis articulating along a multitude of planes is shown in WO04/008987. WO04/008987 describes a dynamic ankle orthesis allowing a rotation along the vertical axis of the leg, and a rotation along the longitudinal axis of the foot. A mortar situated behind the heel enforces a pressure, which causes a lift at the front part of the foot.

The document WO99/66868 describes a dynamic ankle orthesis, which allows a rotation around the longitudinal axis of the foot and a rotation along the vertical axis of the leg. A system of elastic tensioners enforces traction for lifting the front part of the foot.

The last two documents provide rotation around several axes, and assisting dynamically the lifting of the foot.

### Summary of the invention

It is an object of the present invention to provide an improved dynamic ankle orthesis, to be worn on a foot.

It is an object of embodiments of the present invention to harmonize the different phases in making a step. This harmonization of the different consecutive phases improves the stability of the patient and has a direct influence on the dynamic behavior of the whole body of the patient. Amongst others, it permits to ease the articulations of knees, hips and rachis, all promoting a better energetic balance.

The above objective is accomplished by a dynamic ankle orthesis according to the present invention. The orthosis allows control and assistance of the foot during rotation around each of the three axes of rotation in a distinct way during each of the phases of a step.
A dynamic ankle orthesis as subject of the present invention comprises a shoe insert for receiving a foot and having a heel, a cuff for fitting the lower part of the leg and a resilient or springy means for coupling said shoe insert and said cuff. By "foot" is meant the foot of a human patient or a part of an animal which articulates by means of an articulation rotatable around 3 axes.

The resilient means is coupled at its lower part to the shoe insert by means of a pivot located at the heel of the shoe insert. The term "heel" is to be understood as the part of the shoe insert, intended to be located at the heel part of the foot. This is below the axis of the ankle and behind the plane defined by the ankle axis and the vertical axis of the leg, behind meaning in a direction away from the toes.

According to embodiments of the present invention, the resilient means may comprise at least two posts. The resilient means is coupled to the cuff by means of these at least two posts. These at least two posts may merge at the base part of said resilient means, providing a common base connected to the shoe insert.

According to embodiments of the present invention, the resilient means, e.g. comprising at least two posts, may be slidingly mounted in a sheath at the back of the cuff. This slidingly mounted coupling of the sheath and the resilient means, e.g. by means of at least two posts, may allow a vertical displacement of the cuff and a rotation of the resilient means.

The term "back" of the cuff is to be understood as the part of the cuff, which is adapted to receive the calf of the patient's leg. The back part may be understood as the part of the cuff located behind the plane defined by the vertical axis of rotation of the leg and the axis of rotation of the ankle.

According to embodiments of the present invention, the height of front and rear bearing surfaces of the sheath may be different or may be similar and the backlash between said sheath and said posts is limited by means of set screws or wedges.

According to embodiments of the present invention, the coupling point of the sheath and the cuff is located at an offset out of the plane defined by the longitudinal axis of the foot and the vertical axis of the leg. This offset, being the distance between the centre point of the sheath and the plane defined by the longitudinal axis of the foot and the vertical axis of the leg, additional to the inclusion of an angle α between axis of rotation of the pivot with the longitudinal axis of the foot, causes an angular momentum of the foot around the vertical axis of the leg, in case the lower part of the resilient means is forced backwards during different phases of a step, and especially during the toe-off phase. Due to this backward force, and assisted by the springy character of the resilient means, the upper part of the resilient means is pushed forward, causing an angular momentum having a position vector being substantially the offset of the coupling point of the sheath and the plane.

This angular momentum may limit the rotation of the foot due to the action during the propulsive phase. As such, a control of the rotation of the foot around the vertical axis of the leg can be obtained.

According to embodiments of the present invention, the axis of rotation of the pivot may include an angle with the longitudinal axis of the foot in the range of -45° to 45°. Due to the enforced bend on and the springy nature of the resilient means, and due to the back-to-front (or extension-to-flexion) shifting forces applied by the foot to the shoe insert during the phases of a step, the angle causes an angular momentum of the foot around its longitudinal axis. This results in the foot making a rotation around the longitudinal axis of the foot during consecutive phases of a step, this creating and assisting a supination or pronation of the foot.

According to embodiments of the present invention, the height of front and the rear bearing surfaces of the pivot could be different or similar which makes it possible to support differently the extension or the inflection of the foot or to have an equal effect.

According to embodiments of the present invention, the pivot may be detachable for easy adaptation and adjusting of the orthesis to the requirements of the pathology of the patient.

According to embodiments of the present invention, the lower part of the resilient means may be moveable around the pivot in an open area in the shoe insert. The shape of the open area limits the movement around the pivot.

According to embodiments of the present invention, the open area may be provided with setscrews for limiting the movement around the pivot.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 schematically shows a general three-dimensional view of an orthesis according to an embodiment of the present invention.
Fig. 2 schematically shows a top view of an orthesis according to an embodiment of the present invention during the swing-phase or heel-strike phase.
Fig. 3 schematically shows a top view of an orthesis according to an embodiment of the present invention during the toe-off phase.
Fig. 4 shows a detail view of the pivot and its bearing surfaces.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under, in front of, behind and the like in the description and the claims are used for descriptive purposes and not necessarily for describing relative positions. It is to be understood that the terms so used are interchangeable under appropriate circumstances and that the embodiments of the invention described herein may be capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only, except where explicitly stated otherwise. Thus, the scope of the expression "a device A coupled to a device B" should not be limited to devices or systems wherein one piece of device A is directly connected to a piece of device B. It means that there exists a path between the piece of A and the piece of B which may be a path including other devices or means.

The invention will now be described by a detailed description of several embodiments of the invention. It is clear that other embodiments of the invention are exemplary and can be configured according to the knowledge of persons skilled in the art without departing from the true spirit or technical teaching of the invention, the invention being limited only by the terms of the appended claims.

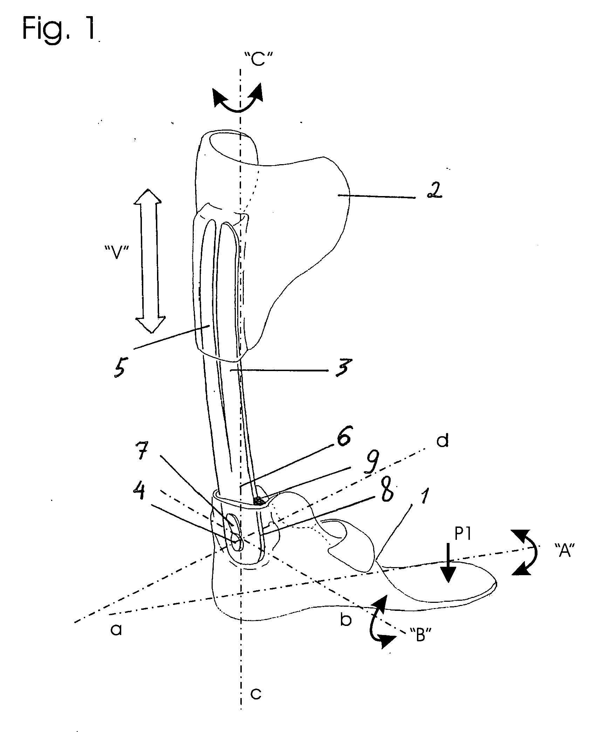

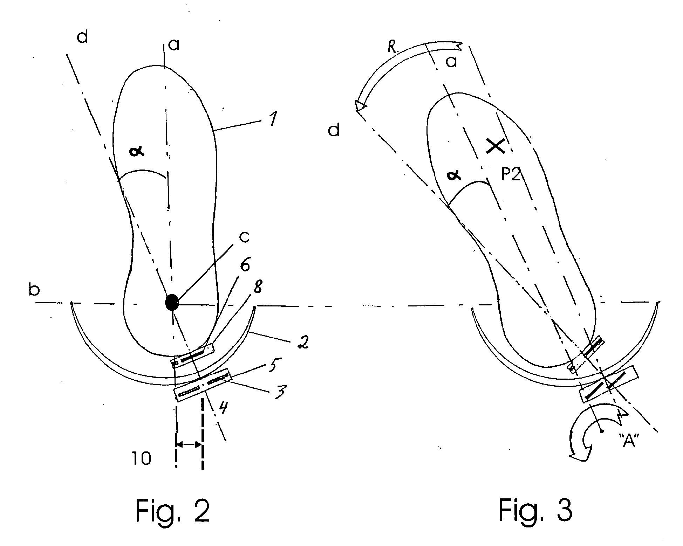

A dynamic orthesis as subject of the present invention will be described hereinafter. The three axes of rotation are shown in Fig. 1 and Fig. 2. The axis "a" is the longitudinal axis of the foot, e.g. a foot of a human patient around which the foot may be rotated, according to arrow "A" in Fig. 1. The axis "b" is axis of the ankle, around which the foot may be rotated according to arrow "B" in Fig. 1. The axis "c" is the vertical axis of rotation of he leg, around which the foot may be rotated according to arrow "C" as shown in Fig. 1. As shown in Fig. 1, means for allowing a displacement "V" in vertical direction between a cuff 2 and posts 3 may be provided.

As is shown in Fig. 1, an embodiment of an orthesis as subject of the present invention comprises three segments, being
● a shoe insert 1 for receiving the foot of a patient,
● a cuff 2 to be fit to the lower part of the leg of the patient, e.g. to fit to the calf of the patient's leg, and
● a resilient or springy means, comprising at least one post, e.g. comprising two posts 3 which have a common base 6, coupling the cuff 2 to the shoe insert 1.

The springy means is coupled at its lower part, e.g. at the level of the common based 6, to the shoe insert 1 by means of a pivot 4 located at the heel of the shoe insert 1.

Preferably, the pivot is located in the lower part of the heel, this is the zone lower than the axis of the ankle be defined, e.g. a zone between halfway the height of the ankle axis and the ground level, which has the advantage that the pivot can be located in the shoe.

Such type of orthesis allows controlling and assisting in a smooth way the rotation "B", this is either bending or stretching, of the ankle around the axis of the ankle (b). The possibility to vary the section, the exact form and the number of posts 3 of the springy means may change the degree of assistance and control given to the bending and stretching of the ankle. By bending the posts 3, the balance point of stretching or bending around the axis of the ankle (b) may be set, which enables the orthesis to be adapted according to the spasticity and muscle tonicity of the foot of the patient.

The upper part of the posts 3, or more in general the upper part of the springy means, are mounted slidingly in vertical direction in a sheath 5 located at the back of the cuff 2, allowing a displacement "V". At the heel of the shoe insert 1, the springy means is coupled to the shoe insert 1 by means of a pivot 4. The height of front and the rear bearing surfaces of the pivot could be different or similar for supporting differently or equally the extension or the inflection of the foot.

The posts 3 may be formed from any suitable resilient or springy material, e.g. metallic or composite materials. They may have one common base at their lower part 6, or may be present as separate posts. The plurality, e.g. two, posts 3 may be replaced by one support, which increases the rigidity around the vertical axis (c) of the leg. The posts 3 may be unitary pieces, i.e. provided in only one part, having a given thickness, or they may be provided as a stack of several layers, each layer having its own thickness and the plurality of layers not all having the same dimensions.

The shoe insert 1 may be formed from any suitable material, which is e.g. strong enough to resist the forces, exercised, such as thermo-formable material or laminated material. This shoe insert 1 has as function to fit to and guide the foot wearing the orthesis.

At the heel of the shoe insert 1, the springy means, e.g. by means of its lower part 6 such as e.g. the common part of the two posts 3, is coupled to the shoe insert 1 by means of a pivot 4. The axis of rotation (d) of the pivot 4 includes an angle α with the longitudinal axis of the foot (a) in the range of -45° to 45°. A negative angle means an axis of rotation (d) inclined to the inner side of the foot as compared to the longitudinal axis of the foot (a), a positive angle is to be understood as inclined to the outer side of the foot. To obtain such negative or positive angle, the point where the springy means is coupled to the heel may be shifted inwards or outwards on the periphery of the heel part of the shoe insert. The angle α between the axis of rotation (d) of the pivot 4 and the longitudinal axis (a) of the foot is measured by measuring the angle between the perpendicular projections of the two axes on a plane perpendicular to the vertical axis (c) of the leg, the projection being made according to the vertical axis (c) of the leg.

An axis of rotation (d) including an angle α of between 20° and 25° as compared to the longitudinal axis (a) of the foot was found to provide very good results. As shown in Fig. 2 and Fig. 3, the axis of rotation (d) of the pivot 4 including an angle α with the longitudinal axis (a) of the foot, causes the foot to make a rotation "A" around the longitudinal axis (a) of the foot during consecutive phases of a step, as indicated with arrow "A" in Fig. 3

During the swing-phase, the foot, e.g. a spastic or dangling foot, exercises a downward pressure to the front part of the shoe insert 1. This pressure (P1) provides to the orthesis an extension couple. Due to this extension couple and due to the orientation of the axis (d) of the pivot 4, this pressure results in causing a supination of the shoe insert 1 and thus of the foot wearing the shoe insert 1. This supination causes the postero external zone of the heel to hit the ground at start of the heel-strike phase.

During the heel-strike phase, the vertical position of the leg is located behind the axis of the heel, which increases further the extension couple of the orthesis. The maximum extension couple, and thus the maximum of supination is obtained immediately after the heel strike. During the heel strike phase and the mid stance, the vertical position of the leg is changed from a position behind the axis of the heel towards the front of the front of the foot, depending on the displacement of the gravity centre of the body from the back to the front. The extension couple is gradually changed to a flexion couple.

This bending couple can be seen as an upwards pressure (P2) on the sole, which on its turn cause a pronation as indicated "A" in Fig. 3, around the longitudinal axis (a) of the foot. As a result, during the toe-off phase, an optimal propulsive push off at the metatarsus of the big toe is obtained. The more correct location of the push off also causes a more efficient dynamic release and use of the energy stored in the resilient means due to its bending resistance and the enforcement of a flexion couple during mid stance.

If necessary, the rotation around the longitudinal axis (a) of the foot may be limited by the shape of an open area 8 in the shoe insert 1, in which the lower part 6 of the springy means is moveable. Possibly the open area 8 is provided with setscrews 9, for limiting the movement around the pivot 4.

The pivot 4 may be easily detachable for allowing easy setting of the axis during adjustment of the orthesis to the pathology of the user. The pivot 4 may comprise a detaching means 7 such as a screw or alike to enable a quick and easy detachment of the shoe insert 1 from the springy means, e.g. the at least one post 3.

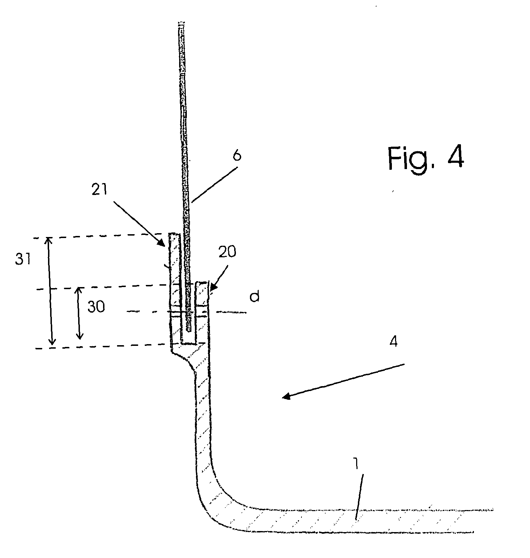

As shown in Fig. 4, the pivot 4 is provided with front bearing surface 20 and the rear bearing surface 21 which front and rear bearing surface have different heights, as indicated in Fig 4 by 30 and 31. This has the advantage that the support given during the extension or the inflection of the foot is different, and can be adjusted according to the specific needs.

In the upper part of the orthesis according to embodiments of the present invention, the cuff 2 may be posterior or antiposterior and may include a condyle fixation. The closing of the cuff 2 may be carried out by any appropriate means, such as straps for example.

The cuff 2 is provided at its back with a sheath 5 in which the upper part of the springy means, e.g. the upper part of the posts 3, is slidingly mounted in vertical direction, allowing a vertical displacement "V". The latter is to limit the pumping effect of the orthesis. The slidingly mounted coupling of the upper part of the springy means, such as e.g. the posts 3, and the sheath 5 allows a vertical displacement of the cuff 2 and may allow rotation of upper part of the springy means, e.g. the posts 3 rotating around their individual axes. A slight pressure on the surfaces of the sheath 5 avoids any extensive thumping of the orthesis.

The height of front and rear bearing surfaces of the sheath 5 may be different to provide different support during flexion or stretching action of the orthesis.

The coupling point of the sheath 5 and the cuff 2 is located at an offset 10 out of the plane defined by the longitudinal axis (a) of the foot and the vertical axis (c) of the leg, which causes an angular momentum of the foot around the vertical axis (c) of the leg during the toe-off phase of a step. It is understood that this coupling point is located at the periphery of the cuff 2 at the back of the cuff 2.

The distance or lateral displacement between the coupling point of the cuff 2 and sheath 5 and the plane, defined by the longitudinal axis (a) of the foot and the vertical axis (c) of the leg, may influence the rotation "C" of the foot around the vertical axis (c) of the leg.

During the toe-off phase of a step, a correct orientation of the foot is an important issue. As shown in Fig. 2 and Fig. 3, a dislocation out of the plane defined by the vertical axis (c) of the leg and the longitudinal axis of the foot of the coupling of the cuff 2 and sheath 5 outwards on the periphery of the cuff 2, additional to the inclusion of an angle α between axis of rotation (d) of the pivot 4 with the longitudinal axis (a) of the foot, causes an inwards angular momentum (in Fig. 3 indicated "R") of the foot around the vertical axis (c) of the leg. The lower part of the springy means is forced backwards during the toe-off phase. Due to this backward force, the upper part of the springy means is pushed forward, causing an inwards angular momentum having a position vector being substantially equal to the offset 10 of the coupling point of the sheath 5 and the plane.

A dislocation of the coupling point of the heel part of the shoe insert 1 with the springy means from a point on the heel part of the shoe insert 1 defined by the plane defined by the longitudinal axis (a) of the foot and the axis (c) of the leg, towards the outer side of the heel part, as also shown in Fig. 2 and Fig. 3, has been found to be advantageous.

The inwards angular momentum caused by the dislocation out of the plane defined by the vertical axis (c) of the leg and the longitudinal axis of the foot of the coupling of the cuff 2 and sheath 5 outwards on the periphery of the cuff 2, limits the outwards rotation of the foot due to the action during the toe-off phase. As such, a control of the rotation "C" of the foot around the vertical axis (c) of the leg can be obtained.

An offset 10 or dislocation in the range of 15 mm to 25 mm of the sheath 5 on the cuff periphery shows a good effect on controlling the rotation of the foot inward and outward around the axis (c) of the leg.

The backlash allowed between the sheath 5 and the springy means, e.g. the posts 3, will influence the amplitude of the rotation "C" according to the vertical axis (c) of the leg. Possibly the amplitude of the rotation is limited by means of setscrews or wedges.

The adjustment of the direction of the axis of pivot 4, as well as the location of the coupling of the shoe-insert 1, springy means and cuff 2, cause a dynamic effect influencing rotation of the foot according to its three axes of rotation. As an example for patients having an internal rotation of foot, the offset and the orientation of the centre point of the sheath should be fixed at the opposite position as the one explained above.

It is to be understood that although preferred embodiments, specific constructions and configurations, as well as materials, have been discussed herein for devices according to the present invention, various changes or modifications in form and detail may be made without departing from the scope and spirit of this invention.

## Claims

1. A dynamic ankle orthesis comprising a shoe insert (1) having a heel, a cuff (2) and a resilient means (3, 6) for coupling said shoe insert (1) and said cuff (2), wherein said resilient means is coupled at its lower part (6) to said shoe insert (1) by means of a pivot (4) located at the heel of said shoe insert (1).

2. A dynamic orthesis as in claim 1, wherein said resilient means (3, 6) comprises at least two posts (3), said resilient means (3, 6) is coupled to the cuff (2) by means of said at least two posts (3).

3. A dynamic orthesis as in claim 2, wherein said at least two posts (3) merge at said lower part (6) of said resilient means (3, 6).

4. A dynamic orthesis as in any one of the claims 1 to 3, wherein said resilient means is slidingly mounted in a sheath (5) at the back of the cuff (2).

5. A dynamic orthesis as in claim 4, wherein said slidingly mounted coupling of said resilient means (3, 6) and said sheath (5) is adapted to allow a vertical displacement (V) of said cuff (2) with respect to said resilient means (3, 6) and a rotation of said springy means (3, 6).

6. A dynamic orthesis as in any one of the claims 4 to 5, wherein the height of front and rear bearing surfaces of the sheath (5) are different and the backlash between said sheath (5) and said resilient means (3, 6) is limited by means of setscrews or wedges.

7. A dynamic orthesis as in any one of the previous claims, there being a coupling point between the cuff (2) and the resilient means (3, 6), wherein said coupling point is located at an offset (10) out of the plane defined by the longitudinal axis (a) of the foot and the vertical axis (c) of the leg.

8. A dynamic orthesis as in any one of the previous claims, wherein the axis of rotation (d) of said pivot (4) includes an angle (α) with the longitudinal axis (a) of the foot in the range of -45° to 45°.

9. A dynamic orthesis as in any one of the previous claims, wherein the pivot (4) is provided with front and the rear bearing surfaces which front and rear bearing surface have different heights

10. A dynamic orthesis as in any one of the previous claims, wherein said pivot (4) is detachable.

11. A dynamic orthesis as in any one of the previous claims, wherein said lower part (6) is moveable around said pivot (4) in an open area (8) in said shoe insert (1), the movement around said pivot (4) being limited by the shape of said open area (8).

12. A dynamic orthesis as in claim 11, wherein said open area (8) is provided with setscrews (9), the movement around said pivot (4) being limited by said setscrews (9).
